(19) Europäisches Patentamt
European Patent Office
Office européen des brevets

(11) Publication number: 0 031 722
B1

(12) EUROPEAN PATENT SPECIFICATION

(45) Date of publication of patent specification: 28.03.84

(51) Int. Cl.³: C 07 H 17/08, A 61 K 31/70

(21) Application number: 80304713.3

(22) Date of filing: 23.12.80

# (54) Water soluble guanidine derivatives of polyene macrolides and the esters thereof, processes for their preparation and pharmaceutical compositions containing them.

(30) Priority: 24.12.79 GB 7944360

(43) Date of publication of application:
08.07.81 Bulletin 81/27

(45) Publication of the grant of the patent:
28.03.84 Bulletin 84/13

(84) Designated Contracting States:
AT BE CH DE FR GB IT LI NL SE

(56) References cited:
BE - A - 884 339
FR - A - 2 150 776

LOUIS F. FIESER: "Reagents for organic synthesis", 1967, John Wiley and Sons Inc., New York, London, Sydney, page 236

JOURNAL OF THE CHEMICAL SOCIETY, Perkin Transactions I, 1974, J. ABERHART et al.: "The constitution of Primycin. Part I. Characterisation, Functional groups, and degradation to the secoprimycins", pages 816, 826

(73) Proprietor: A/S Dumex (Dumex Ltd.)
37, Prags Boulevard Post Box No. 1736
DK-2300 Copenhagen S. (DK)

(72) Inventor: Witzke, Niels
38 B, Pardun Road
North Brunswick New Jersey 08902 (US)

(74) Representative: Watkins, Arnold Jack
European Patent Attorney Frank B. Dehn & Co.
Imperial House 15-19 Kingsway
London WC2B 6UZ (GB)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

# Water soluble guanidine derivatives of polyene macrolides and the esters thereof, processes for their preparation and pharmaceutical compositions containing them

The present invention relates to new guanidine derivatives of amphotericin B and amphotericin B methyl ester and to processes for their preparation.

Amphotericin B possesses the formula:

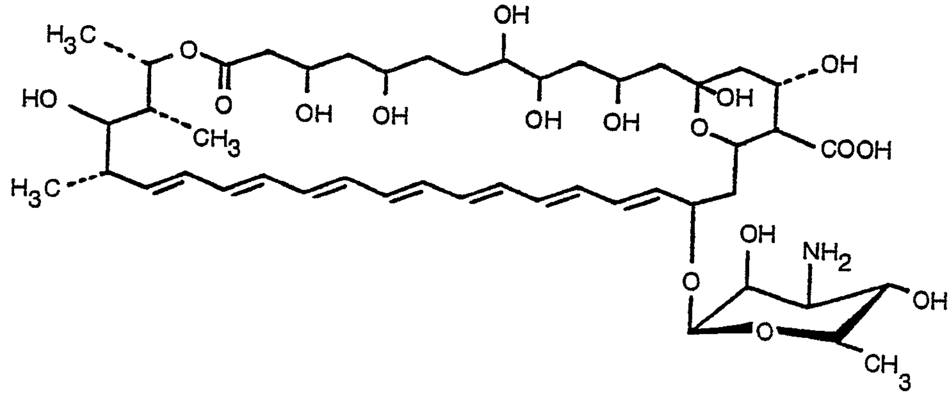

Amphotericin B, which, in spite of its toxicity, hitherto has been the antibiotic of choice in the treatment of systemic mycoses, can only be administered by intravenous injection. The methyl ester of amphotericin B, which is described in U.S. Patent No. 3,945,993, is less toxic, but similarly can only be administered intravenously. The intravenous administration in both cases results in rapid excretion and a correspondingly short period of action. However, the guanidine derivatives of amphotericin B and the methyl ester thereof which are the subject of the present invention, may be injected intramuscularly thus prolonging the beneficial activity of the antibiotic.

According to the present invention there is provided a process for the preparation of a guanidine derivative of amphotericin B or the methyl ester thereof, optionally in the form of a salt, characterized in that amphotericin B or an ester thereof is reacted with a carbodiimide of the formula:—

$$R^I—N{=}C{=}N—R^{II} \qquad I$$

(wherein $R^I$ represents a 3-dimethylaminopropyl group when $R^{II}$ represents an ethyl group or $R^I$ represents a 2-(N-methylmorpholinium)ethyl group when $R^{II}$ represents a cyclohexyl group) or a salt thereof whereby to form a guanidine derivative of amphotericin B or the methyl ester thereof, optionally in the form of a salt and where the guanidine derivative of amphotericin B or a slt thereof is obtained optionally converting said derivative or salt thereof into the methyl ester thereof.

When it is desired to obtain the said guanidine derivative in the form of the methyl ester, the reaction is conveniently effected in the presence of methanol.

The antibiotic amphotericin B used as starting material is insoluble in water at physiological pH, and therefore the guanidine derivatives of the present invention are of considerable interest as antimycotic agents, since they have a good solubility in water. The compounds of the present invention have been found to possess a lower toxicity than amphotericin B when administered intraperitoneally to mice.

It is advantageous to effect the reactions between amphotericin B and the carbodiimides of formula (I) at or below room temperature e.g. 0—10°C, preferably in the presence of N-methylpyrrolidone, hexamethylphosphoric acid amide, or a mixture of hexamethylphosphoric acid amide and tetramethylurea.

Where the compound of formula I is used in the form of a salt, the stabilizing anion may be any convenient anion e.g. a halide such as a chloride or an arylsulphonate such as a toluenesulfonate e.g. a p-toluenesulphonate.

In order to avoid esterification of the carboxylic acid group of the amphotericin B, where this is desired, it is advantageous to carry out the preparation of the guanidine type derivative in the presence of up to 10% of water.

When it is desired to prepare the methyl ester of the guanidine derivative, the reaction between amphotericin B and the carbodiimide of formula (I) is advantageously effected in the presence of an acid e.g. up to one equivalent of an acid, preferably an acid which is not or which is only slowly esterified by methanol, for example an organic sulfonic acid, a Lewis acid or an inorganic acid.

The esterification reaction may for example be effected in the presence of a mixture of methanol and a solvent in which the amphotericin B shows good solubility.

Where it is desired to prepare the methyl ester and amphotericin B is used in the form of the acid as starting material in the preparation of the guanidine derivative, the methyl ester may if desired, be

formed *in situ* by effecting the preparation of the guanidine derivative in the presence of methanol.

According to a further feature of the present invention there is provided a compound of the formula:—

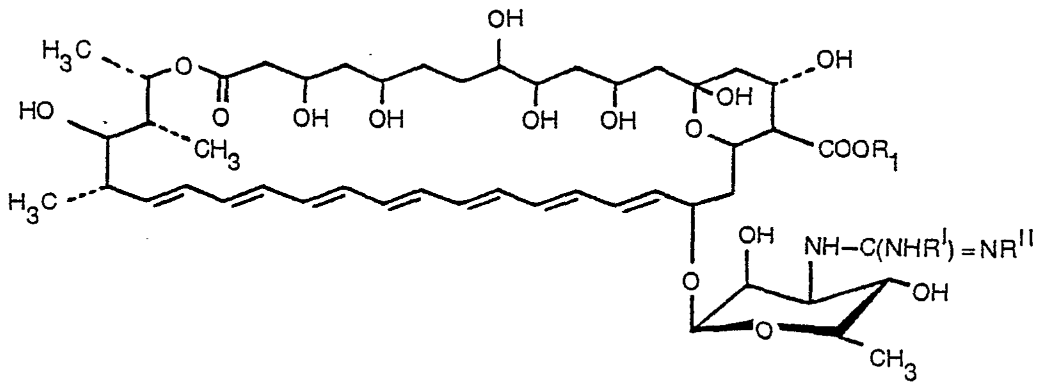

II

(wherein $R^I$ and $R^{II}$ are as hereinbefore defined and $R_1$ represents a hydrogen atom or a methyl group) and the salts thereof.

Thus, for example, compounds of the present invention include:

a) the hydrochloride salt of a compound of formula II wherein $R_1$ represents a hydrogen atom, $R^I$ represents a 3-dimethylaminopropyl group and $R^{II}$ represents an ethyl group;

b) a compound of formula II wherein $R_1$ represents a hydrogen atom, $R^I$ represents 2-(N-methylmorpholinium)-ethyl-*p*-toluenesulfonate group and $R^{II}$ represents a cyclohexyl group in the form of the *p*-toluenesulfonate salt; and

c) a compound of formula II wherein $R_1$ represents a methyl group, $R^I$ represents a 2-(N-methylmorpholinium)-ethyl-*p*-toluenesulfonate group and $R^{II}$ represents a cyclohexyl group in the form of the *p*-toluenesulfonate salt.

According to a further feature of the present invention there are provided pharmaceutical compositions comprising a compound of formula II as hereinbefore defined or a physiologically compatible salt thereof and a carrier.

The guanidine derivatives of the present invention may for example be administered to human patients intravenously or more preferably intramuscularly for the treatment of systemic mycoses. The daily dosage will depend on the condition to be treated, the characteristics of the individual patient and the polyene macrolide used, but will in general be from 1 to 10 preferably 2 to 5 mg/kg body weight per day for intramuscular and intravenous administration.

The guanidine derivatives of the present invention may also be administered orally where no systemic effect is required, for example where an effect is only desired on the intestinal tract. For oral administration a dosage range of from 400 mg to 10 g. preferably 400 mg to 5 g. per day is generally used.

Pharmacological and toxicological test data

Derivative of 1-(3-dimethylaminopropyl-3-ethyl carbodiimide (Formula II: $R^I$ = dimethylaminopropyl, $R^{II}$ = ethyl and $R_1$ = hydrogen) with amphotericin B (DAPEC—AB, minimum fractionary inhibition (MFC) against fungi, compared with amphotericin B (AB).

| Organism | FIC, mcg/ml AB | FIC, mcg/ml DAPEC—AB |
|---|---|---|
| *Candida albicans* 1. | 0.63 | 3.90 |
| *Candida albicans* 2. | 1.30 | 1.30 |
| *Aspergillus niger* | 3.20 | 2.40 |
| *Paecilomyces varioti* | 0.79 | 0.96 |

Comparison of acute toxicities of AB and DAPEC-AB when administered intravenously to mice (CFI, males).

| | Dose, mg/kg | Mortality, 1 day | Time of death |
|---|---|---|---|
| AB | 3.5 | 3/8 | Less than 2 mins |
| | 4.2 | 6/8 | |
| DAPEC—AB | 32 | 1/8 | 1 to 3 hours |
| | 38 | 4/8 | |
| | 44 | 5/8 | |

The invention is illustrated by the following examples:

Example 1

Derivative of 1-(3-dimethylaminopropyl)-3-ethylcarbodiimide with amphotericin B (DAPEC-AB.HCl Formula II; $R^I$ = 3-dimethylaminopropyl, $R^{II}$ = ethyl and $R_1$ = hydrogen)

40 mg of amphotericin B, 0.01 ml of a 20% solution of butylhydroxytoluene in tetrahydrofuran, and 0.018 ml 20% 1-methylimidazole in tetrahydrofuran are dissolved in 1 ml of a mixture of equal parts of hexamethylphosphoric acid amide and tetramethylurea, When all the substances have dissolved 0.1 ml water and 9 mg of 1-(3-dimethylaminopropyl)-3-ethylcarbodiimide hydrochloride are added, and the mixture is stirred for 3—4 days under an atmosphere of argon at 6—8°C. The reaction is monitored by thin layer chromatography (TLC) (see below). The pH should be in the range 6.5—8, and is estimated in 0.025 ml of the reaction mixture diluted with 0.4 ml of argonized water.

The reaction mixture is diluted with 15 ml of acetone, and centrifuged at 6°C. The supernatant is collected, concentrated *in vacuo*, and added to 20 ml of diethyl ether whilst stirring vigorously. The mixture is stirred for 1 hour in an ice box. The precipitate is recovery by filtration and washed with diethyl ether. After drying *in vacuo* over concentrated sulfuric acid, 20 mg of 1-(3-dimethylaminopropyl)-3-ethylcarbodiimide amphotericin B hydrochloride is obtained as a light yellow powder.

*E* 1% (382 nm, MeOH): 1320

*IR*: 1650—1700 $cm^{-1}$: C=N stretching band

no absorption at 1400 $cm^{-1}$: no primary amino group.

*TLC-system*

Tetrahydrofuran and 0.7 M ammonia (4:1)

Silica gel TLC-plates.

Rf: 0.36, the derivative.

Rf: 0.28, amphotericin B.

Example 2

Di-p-toluenesulfonate of derivative of 1-[2-(N-methylmorpholinium)ethyl]-3-cyclohexyl carbodiimide with amphotericin B methyl ester (Formula II: $R^I$ = 2-(N-methyl-N-tosyloxymorpholinium)ethyl, $R^{II}$ = cyclohexyl and $R_1$ = methyl).

40 mg of amphotericin B is dissolved in a mixture of 0.4 ml of hexamethylphosphoric acid amide, 0.1 ml of tetramethylurea and 0.01 ml of a 20% solution of butylhydroxytoluene in tetrahydrofuran. To this solution is added 47 mg of 1-(N-methylmorpholiniumethyl)-3-cyclohexylcarbodiimide *p*-toluenesulfonate, 0.5 ml of anhydrous methanol, and 0.025 ml of 2 M pyridine *p*-toluenesulfonate in methanol. The mixture is stirred at 6—8°C for 17 hours under an atmosphere of argon. A further 10 ml of 1-(N-methylmorpholiniumethyl)-3-cyclohexylcarbodiimide *p*-toluenesulfonate is added, while stirring at 6—8°C under an atmosphere of argon is continued for an additional 54 hours. The reaction is monitored by TLC (see below). The pH was determined by diluting 0.025 ml of the reaction mixture with 0.4 ml of argonized water, and is found to increase from 4.4 to 6.0 during the reaction.

The solution is mixed with 5 ml of acetone, centrifuged at 6°C, and 0.002 ml tributylamine is added to the supernatant, followed by 12 ml of dry ethyl ether; the precipitate is centrifuged and washed twice with 10 ml of ethyl ether. After drying *in vacuo* over concentrated sulfuric acid, 30 mg of 1-(N-methylmorpholiniumethyl)-3-cyclohexylcarbodiimide amphotericin B methylester di-*p*-toluenesulfate is obtained.

The $H^1$-NMR ($d_6$-DMSO) shows a singlet at $\delta = 3.7$ ppm ($-COOCH_3$).
*IR*: 1650—1700 $cm^{-1}$: C=N stretching band
no absorption at 1400 $cm^{-1}$: no primary amino group.
*TLC-system*
Tetrahydrofuran, methanol and 5% aqueous ammonium carbonate (3:1:1).
Silica gel TLC-plate.
Rf: 0.78 amphotericin methylester.
Rf: 0.30 amphotericin B.
Rf: 0.13 the guanidine type derivative methyl ester.
Rf: 0.06 the guanidine type derivative.

**Claims**

1. A process for the preparation of a guanidine derivative of amphotericin B or the methyl ester thereof, optionally in the form of a salt, characterized in that amphotericin B or an ester thereof is reacted with a carbodiimide of the formula:—

$$R^I—N=C=N—R^{II} \qquad I$$

(wherein $R^I$ represents a 3-dimethylaminopropyl group when $R^{II}$ represents an ethyl group or $R^I$ represents a 2-(N-methylmorpholinium)ethyl group when $R^{II}$ represents a cyclohexyl group) or a salt thereof whereby to form a guanidine derivative of amphotericin B or the methyl ester thereof, optionally in the form of a salt and where the guanidine derivative of amphotericin B or a salt thereof is obtained optionally converting said derivative or salt thereof into the methyl ester thereof.

2. A process as claimed in claim 1 for the preparation of the methyl ester of the guanidine derivative of amphotericin B or a salt thereof wherein the reaction is effected in the presence of methanol.

3. A compound of the formula:—

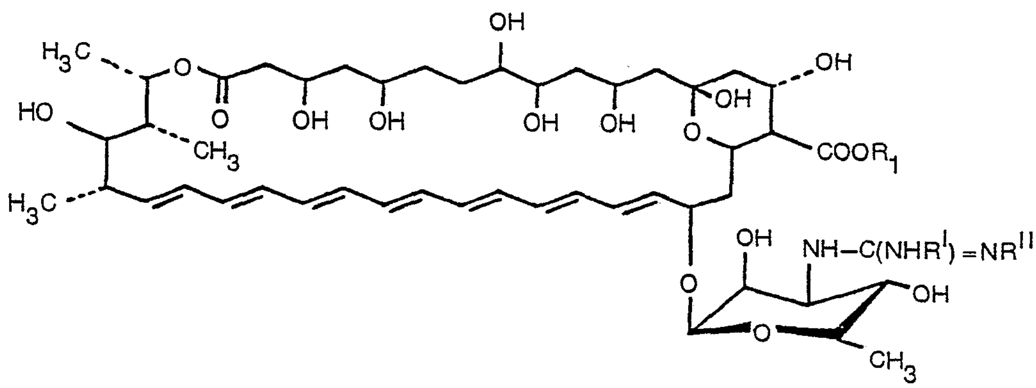

II

(wherein $R^I$ and $R^{II}$ are as hereinbefore defined in claim 1 and $R_1$ represents a hydrogen atom or a methyl group) and the salts thereof.

4. A compound as claimed in claim 3 wherein $R_1$ represents a hydrogen atom, $R^I$ represents a 3-dimethylaminopropyl group and $R^{II}$ represents an ethyl group in the form of its hydrochloride salt.

5. A compound as claimed in claim 3 wherein $R_1$ represents a hydrogen atom, $R^I$ represents a 2-(N-methylmorpholinium)ethyl-*p*-toluenesulfonate group and $R^{II}$ represents a cyclohexyl group in the form of the *p*-toluenesulfonate salt.

6. A compound as claimed in claim 3 wherein $R_1$ represents a methyl group, $R^I$ represents a 2-(N-methylmorpholinium)ethyl-*p*-toluenesulfonate group and $R^{II}$ represents a cyclohexyl group in the form of the *p*-toluenesulfonate salt.

7. Pharmaceutical compositions comprising a compound of formula II as defined in any one of claims 3 to 6 or a physiologically compatible salt thereof and a carrier.

**Revendications**

1. Un procédé de préparation d'un dérivé de guanidine de l'amphotéricine B ou de son ester méthylique, éventuellement sous forme d'un sel, caractérisé en ce qu'on fait réagir l'amphotéricine B ou un de ses esters avec un carbodiimide de formule:

$$R^I—N=C=N—R^{II} \qquad I$$

(dans laquelle $R^I$ représente un groupe 3-diméthylaminopropyle lorsque $R^{II}$ représente un groupe éthyle, ou

$R^I$ représente un groupe 2-(N-méthylmorpholinium)éthyle lorsque $R^{II}$ représente un groupe cyclohexyle) ou un de ses sels pour former un dérivé de guanidine de l'amphotéricine B ou de son ester méthyliqué, éventuellement sous forme d'un sel et, lorsque l'on obtient le dérivé de guanidine de l'amphotéricine B ou un de ses sels, on transforme éventuellement ledit dérivé ou son sel en l'ester méthylique.

2. Un procédé suivant la revendication 1 pour la préparation de l'ester méthylique du dérivé de guanidine de l'amphotéricine B ou d'un de ses sels dans lequel on effectue la réaction en présence de méthanol.

3. Un composé de formule

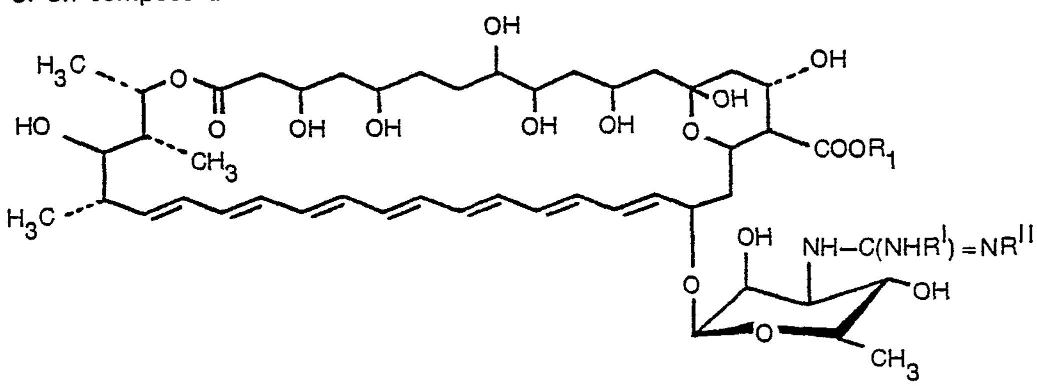

II

(dans laquelle $R^I$ et $R^{II}$ sont tels que définis à la revendication 1 et $R_1$ représente un atome d'hydrogène ou un groupe méthyle) et ses sels.

4. Un composé suivant la revendication 3, dans lequel $R_1$ représente un atome d'hydrogène, $R^I$ représente un groupe 3-diméthylaminopropyle et $R^{II}$ représente un groupe éthyle, sous forme de son chlorhydrate.

5. Un composé suivant la revendication 3, dans lequel $R_1$ représente un atome d'hydrogène, $R^I$ représente un groupe 2-(N-méthylmorpholinium)éthyl-p-toluènesulfonate et $R^{II}$ représente un groupe cyclohexyle, sous la forme du p-toluènesulfonate.

6. Un composé suivant la revendication 3, dans lequel $R_1$ représente un groupe méthyle, $R^I$ représente un groupe 2-(N-méthylmorpholinium)éthyl-p-toluènesulfonate et $R^{II}$ représente un groupe cyclohexyle, sous la forme du p-toluènesulfonate.

7. Compositions pharmaceutiques comprenant un composé de formule II comme défini à l'une quelconque des revendications 3 à 6 ou un de ses sels physiologiquement compatibles et un véhicule.

## Patentansprüche

1. Verfahren zur Herstellung eines Guanidinderivats von Amphotericin B oder des Methylesters davon, gegebenenfalls in Form eines Salzes, dadurch gekennzeichnet, daß man Amphotericin B oder einen Ester davon mit einem Carbodiimid der Formel:

$$R^I—N=C=N—R^{II} \qquad I$$

(worin $R^I$ eine 3-Dimethylaminopropylgruppe bedeutet, wenn $R^{II}$ eine Ethylgruppe bedeutet oder $R^I$ eine 2-(N-Methylmorpholinium)ethylgruppe bedeutet, wenn $R^{II}$ eine Cyclohexylgruppe bedeutet) oder einem Salz davon zur Reaktion bringt, wodurch ein Guanidinderivat von Amphotericin B oder der Methylester davon, gegebenenfalls in Form eines Salzes, gebildet wird und wenn das Guanidinderivat von Amphotericin B oder ein Salz davon erhalten wird, gegebenenfalls dieses Derivat oder ein Salz davon in den Methylester davon überführt wird.

2. Verfahren gemäß Anspruch 1 zur Herstellung des Methylesters des Guanidinderivats von Amphotericin B oder eines Salzes davon, dadurch gekennzeichnet, daß die Reaktion in Gegenwart von Methanol durchgeführt wird.

3. Eine Verbindung der Formel

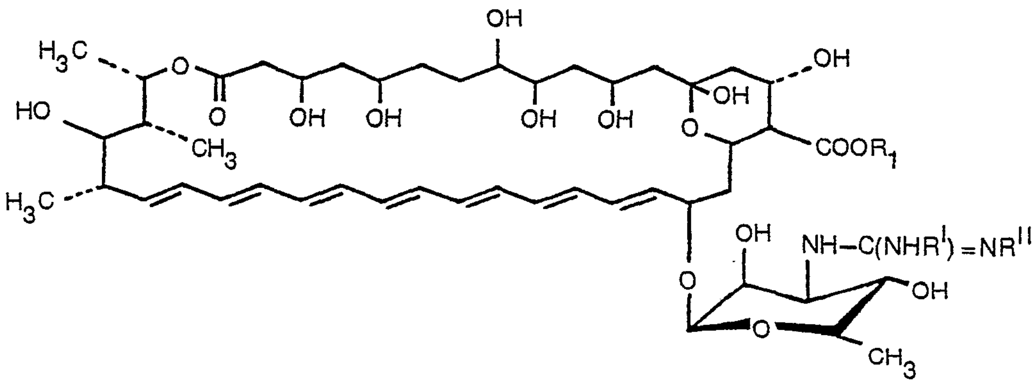

II

worin $R^I$ und $R^{II}$ wie in Anspruch 1 definiert sind und $R_1$ ein Wasserstoffatom oder eine Methylgruppe bedeutet) und dessen Salze.

4. Eine Verbindung wie in Anspruch 3 beansprucht, worin $R_1$ ein Wasserstoffatom bedeutet, $R^I$ eine 3-Dimethylaminopropylgruppe ist und $R^{II}$ eine Ethylgruppe ist, in Form von dessen Hydrochloridsalz.

5. Eine Verbindung wie in Anspruch 3 beansprucht, worin $R_1$ ein Wasserstoffatom bedeutet, $R^I$ eine 2-(N-Methylmorpholinium)-ethyl-p-toluolsulfonatgruppe darstellt und $R^{II}$ eine Cyclohexylgruppe bedeutet, in Form des p-Toluolsulfonatsalzes.

6. Eine Verbindung wie in Anspruch 3 beansprucht, worin $R_1$ eine Methylgruppe bedeutet, $R^I$ eine 2-(N-Methylmorpholinium)-ethyl-p-toluolsulfonatgruppe darstellt und $R^{II}$ eine Cyclohexylgruppe bedeutet, in Form des p-Toluolsulfonatsalzes.

7. Pharmazeutische Zusammensetzungen umfassend eine Verbindung der Formel II wie in einem der Ansprüche 3 bis 6 definiert oder ein physiologisch verträgliches Salz davon und einen Träger.